**Europäisches Patentamt**

**European Patent Office**

**Office européen des brevets**

(19)

(11) Veröffentlichungsnummer: **0 301 343 B1**

(12) # EUROPÄISCHE PATENTSCHRIFT

(45) Veröffentlichungstag der Patentschrift:
21.08.91 Patentblatt 91/34

(51) Int. Cl.$^5$: **C07C 25/08, C07C 17/00**

(21) Anmeldenummer: **88111499.5**

(22) Anmeldetag: **18.07.88**

(54) **Verfahren zur Herstellung von 1,2-Dichlorbenzol.**

(30) Priorität: **30.07.87 DE 3725196**

(43) Veröffentlichungstag der Anmeldung:
**01.02.89 Patentblatt 89/05**

(45) Bekanntmachung des Hinweises auf die Patenterteilung:
**21.08.91 Patentblatt 91/34**

(84) Benannte Vertragsstaaten:
**DE FR GB IT**

(56) Entgegenhaltungen:
**US-A- 2 866 828**

(73) Patentinhaber: **BAYER AG**
**W-5090 Leverkusen 1 Bayerwerk (DE)**

(72) Erfinder: **Käsbauer, Josef, Dr.**
**Gartenfeld 37**
**W-5632 Wermelskirchen (DE)**
Erfinder: **Fiege, Helmut, Dr.**
**Walter-Flex-Strasse 23**
**W-5090 Leverkusen (DE)**
Erfinder: **Schmidt, Herbert**
**Im Hederichsfeld 52**
**W-5090 Leverkusen 3 (DE)**
Erfinder: **Wedemeyer, Karlfried, Dr.**
**Bilharzstrasse 7**
**W-5000 Köln 80 (DE)**

## Beschreibung

Die vorliegende Erfindung betrifft ein Verfahren zur katalytischen Hydrodehalogenierung von 1,2,4-Trichlorbenzol mit Wasserstoff zu 1,2-Dichlorbenzol an einem Platin/Al-Spinell-Katalysator.

Aus US 2.866.828 ist es bekannt, trihalogenierte Benzole an verschiedenen Katalysatoren zu enthalogenieren. Hierbei wird vornehmlich das 1,3-Dihalogenbenzol angestrebt. In Beispiel 4 dieses US-Patents wird ein 1,2,4-trisubstituiertes Benzol an einem Platin/Kohle-Katalysator enthalogeniert. Hierbei wird jedoch in nur einem Kurzzeitversuch das technisch uninteressante 1,2,4-Tribrombenzol eingesetzt; zudem bildet sich ein hoher Anteil an Monobrombenzol.

US 2.943.114 beschreibt ebenfalls anhand von Kurzzeitversuchen die Herstellung von m-Dichlorbenzol. Bei Benutzung verschiedener Katalysatoren wird insgesamt eine nur schwach ausgeprägte Selektivität der Enthalogenierung beobachtet. Im Beispiel 5 dieses US-Patents wird an einem Platin/Aluminiumoxid-Katalysator ohne Mitteilung des Versuchsergebnisses gearbeitet.

In US 3.595.931 wird mit Hilfe eines mit Kaliumhydroxid dotierten Platin/Aluminiumoxid-Katalysators eine vollständige Enthalogenierung angestrebt, die laut Beispiel 5 dieses US-Patents zu 70% gelingt.

US 2.886.605 beschreibt die Enthalogenierung von aliphatischen bzw. aromatischen Halogenverbindungen. Gearbeitet wird in der Wirbelschicht an einem Aluminiumoxid/Kupfer-(I)-chlorid-Katalysator. Nach Aussage der beschriebenen Kurzzeitversuche erhält man im Reaktionsgemisch nur wenig Dichlorbenzole, deren Hauptanteil das m-Isomere ist.

Für die Durchführung einer Hydrodehalogenierung unter industriellen Aspekten ist jedoch eine hohe Selektivität des Verfahrens und eine hohe Standzeit des Katalysators bei nahezu unverminderter Aktivität gewünscht. Überraschend wurde nun gefunden, daß hierbei die Auswahl von Spinellen als Trägermaterialien die überragende Rolle spielt. Solche erfindungsgemäß eingesetzten Katalysatoren zeigen auch in Dauerversuchen bei über 2.000 Stunden nur eine geringe Desaktivierung.

Es wurde ein Verfahren zur Herstellung von 1,2-Dichlorbenzol gefunden, das dadurch gekennzeichnet ist, daß man 1,2,4-Trichlorbenzol als Einsatzmaterial in der Gasphase bei einer Temperatur vom Siedepunkt des Einsatzmaterials bis 400°C mit Wasserstoff an einem Platin/Al-Spinell-Katalysator umsetzt.

1,2,4-Trichlorbenzol kann beispielsweise durch Chlorieren von 1,4-Dichlorbenzol in einer dem Fachmann geläufigen Weise erhalten werden.

Erfindungsgemäß kann das 1,2,4-Trichlorbenzol auch als Gemisch mit anderen Polychlorbenzolen eingesetzt werden. Hierunter sind andere Trichlorbenzole und Tetrachlorbenzole zu verstehen, bevorzugt das 1,2,3-Trichlorbenzol, das 1,2,3,4-Tetrachlorbenzol und das 1,2,4,5-Tetrachlorbenzol. Auch untergeordnete Mengen von Dichlorbenzolen, insbesondere das gewünschte 1,2-Dichlorbenzol kann im Einsatzmaterial neben 1,2,4-Trichlorbenzol vorliegen. Diese breite Anwendbarkeit des erfindungsgemäßen Verfahrens auch auf technische Chlorierungsgemische ist von besonderem Vorteil. Insbesondere kann ein Chlorierungsgemisch mit einem Gehalt an 1,2,4-Trichlorbenzol eingesetzt werden, das aus der Chlorierung von 1,4-Dichlorbenzol entstanden ist, ohne daß ein solches Gemisch auf reines 1,2,4-Trichlorbenzol aufgearbeitet zu werden braucht.

Erfindungsgemäß werden Platin/Al-Spinell-Katalysatoren eingesetzt. Das Platin liegt hierbei in einer Menge von 0,1 bis 5 Gew.-%, bevorzugt 0,5-2 Gew.-%, bezogen auf das Gesamtgewicht des Katalysators, vor. Eine höhere Platinkonzentration bringt keinen weiteren Vorteil. Der Träger des Katalysators ist ein Al-Spinell. Als Spinellbildende Elemente kommen beispielsweise Li, Be, Mg, Zn, Mn, Cu, Ni in Betracht. In besonderer Weise sei als Trägermaterial LiAl-Spinell genannt.

Es wurde ferner gefunden, daß der beschriebene Katalysator eine weitere Verbesserung für das erfindungsgemäße Verfahren erfährt, wenn er zusätzlich mit einem Erdalkali(hydr)oxid dotiert ist. Die Erdalkalimetalle können hierbei Mg, Ca, Sr oder Ba, sowie Gemische von ihnen, sein. Bevorzugt ist eine Dotierung mit Magnesium(hydr)oxid. Eine solche Dotierung liegt in einer Menge von 0,01 bis 10 Gew.-%, bevorzugt 0,1 bis 5 Gew.-%, bezogen auf das Gesamtgewicht des Katalysators, vor.

Zur Herstellung des erfindungsgemäß eingesetzten Katalysators wird ein käuflicher Al-Spinell, beispielsweise LiAl-Spinell, mit einer wäßrigen Platinsalzlösung getränkt und mit einer Base (Natronlauge, Kalilauge, Natriumcarbonatlösung, Kaliumcarbonatlösung u.a.) und einem Reduktionsmittel (Hydrazinhydrat, Ameisensäure, Formaldehyd u.a.) zur Ausfällung von metallischem Platin behandelt. Anschließend wird ein solcher Katalysator gewaschen und bei erhöhter Temperatur, beispielsweise 300 bis 500°C, getrocknet. Eine solche Trocknung kann vorteilhaft in dem für die erfindungsgemäße Hydrodehalogenierung vorgesehenen Reaktionsapparat vor dem Zusatz des Einsatzmaterials und des Wasserstoffs vorgenommen werden. Für den Fall, daß der erfindungsgemäß einzusetzende Katalysator zusätzlich mit einem Erdalkalimetall(hydr)oxid dotiert werden soll, wird eine geeignete Erdalkalisalzlösung (beispielsweise ein Chlorid oder ein Nitrat), vorteilhaft gemeinsam mit der Platinsalzlösung, auf den Al-Spinell aufgetränkt. In einer dem Fachmann bekannten Weise wird die Menge und die Konzentration der das Platin bzw. das Erdalkalisalz enthaltenden Lösung so gewählt, daß die gewünschte Endkonzentration erreicht wird. Bei der Behandlung mit einer Base wird sodann das Erdalkalimetall in Form seines Hydroxids abgeschieden. Bei der Trocknung im angegebenen Temperaturbereich kann angenommen werden, daß das Erdalkalimetallhydroxid teilweise in das Erdalkalioxid übergeht.

Die Hydrodehalogenierung (Enthalogenierung) wird in der Gasphase in beliebigen, hierzu geeigneten Reaktionsapparaturen durchgeführt. Die Temperatur für das erfindungsgemäße Verfahren kann vom Siedepunkt des Einsatzmaterials bis 400°C, bevorzugt von 250 bis 350°C, gewählt werden.

Die Umsetzung kann sowohl bei Normaldruck als auch bei erhöhtem Druck, beispielsweise bis zu 10 bar, durchgeführt werden, wobei die Temperatur so eingestellt wird, daß das Einsatzmaterial in der Gasphase vorliegt.

Wasserstoff wird in einer Menge von 0,5 bis 5 Mol, bevorzugt 0,9 bis 2 Mol pro Äquivalent des zu entfernenden Chlors eingesetzt. Hierdurch kann der gewünschte Umsatz im erfindungsgemäßen Verfahren gewählt werden. In bevorzugter Weise wird Wasserstoff im Überschuß eingesetzt.

Beispiel 1

LiAl-Spinell wurde mit einer wäßrigen Pt-Salzlösung getränkt, mit Natronlauge und Hydrazinhydrat behandelt und anschließend gewaschen. 35 ml Katalysator mit einer Körnung von 1,5 bis 2,5 mm wurden in einem beheizbaren Glas- oder Metallrohr bei 300 bis 500°C getrocknet und dehydratisiert. Der Katalysator enthielt 1 Gew.-% Pt, bezogen auf sein Gesamtgewicht.

Bei 300°C wurden stündlich 19 ml 1,2,4-Trichlorbenzol verdampft und im dampfförmigen Zustand am Katalysator mit 4 l $H_2$/h umgesetzt. Das Produktgemisch wurde kondensiert und gaschromatographisch untersucht. Aus dem Dauerversuch sind drei exemplarische Zusammensetzungen aufgeführt.

| Substanz [%] | Standzeit [h] | | |
|---|---|---|---|
| | 52 | 221 | 841 |
| Benzol | 7,6 | 4,0 | 3,2 |
| Chlorbenzol | 5,1 | 5,3 | 6,1 |
| 1,2-Dichlorbenzol | 27,6 | 26,5 | 25,1 |
| 1,3-Dichlorbenzol | 4,9 | 4,9 | 4,9 |
| 1,4-Dichlorbenzol | 1,7 | 2,3 | 2,5 |
| 1,2,4-Trichlorbenzol | 53,0 | 56,9 | 57,9 |

Der Katalysator wurde während der gesamten Reaktionszeit nicht regeneriert.

Beispiel 2

Der gleiche Katalysator wie in Beispiel 1, jedoch zusätzlich mit 1% MgO dotiert, wurde im Dauerversuch bei 300°C eingesetzt. Bei einer stündlichen Durchsatzmenge von 19 ml 1,2,4-Trichlorbenzol und 4 l $H_2$ ergab sich nach 97 Stunden Laufzeit das in der Tabelle aufgeführte Produktgemisch (1. Spalte).

Die Werte in der zweiten Spalte beziehen sich auf einen Durchsatz von 35 ml 1,2,4-Trichlorbenzol pro Stunde nach 100 h Laufzeit. Dieser Versuch lief ca. 1000 Stunden ohne Regenerieren bei unveränderter Katalysatoraktivität.

|  | 97 h bei<br>19 ml/h | 100 h bei<br>35 ml/h |
|---|---|---|
| Benzol | 4,3 % | 1,6 % |
| Chlorbenzol | 12,9 % | 5,9 % |
| 1,2-Dichlorbenzol | 36,5 % | 22,4 % |
| 1,3-Dichlorbenzol | 6,7 % | 4,9 % |
| 1,4-Dichlorbenzol | 3,8 % | 3,2 % |
| 1,2,4-Trichlorbenzol | 35,8 % | 59,0 % |

Beispiel 3

Der gleiche Katalysator wie in Beispiel 2 wurde in einem Druckrohr bei 4 bar/300°C eingesetzt. Es wurden 60 ml 1,2,4-Trichlorbenzol und 10,8 l $H_2$ pro Stunde an 30 ml Katalysator umgesetzt. Im Rahmen eines Dauerversuches wurde nach 101 Stunden folgendes repräsentatives Produktspektrum erhalten :

| Benzol | 2,6 |
|---|---|
| Chlorbenzol | 8,1 |
| 1,2-Dichlorbenzol | 26,0 |
| 1,3-Dichlorbenzol | 5,6 |
| 1,4-Dichlorbenzol | 3,7 |
| 1,2,4-Trichlorbenzol | 54,0 |

Der Versuch lief 1500 Stunden ohne Regenerierung bei nahezu unveränderter Katalysatoraktivität und Selektivität.

Beispiel 4-6 (Vergleichsbeispiele)

Im Vergleich zu den Beispielen 1 und 2 wurde 1% Pt auf andere Trägermaterialien aufgebracht und unter den gleichen Bedingungen untersucht. Die folgende Tabelle zeigt den Umsatz nach 50 und 100 Stunden im Dauerversuch.

| Bei-<br>spiel | Trägermaterial | Umsatz (%)<br>nach<br>50 h | 100 h | |
|---|---|---|---|---|
| 4 | $SiO_2$ | 13±1 | 9±1 | Vergleichsbeispiel |
| 5 | Kohle | 15±1 | 11±1 | Vergleichsbeispiel |
| 6 | $Al_2O_3$ | 21±1 | 16±3 | Vergleichsbeispiel |
| 1 | LiAl-Spinell | 45±3 | 38±3 | |
| 2 | MgO/LiAl-Spinell | 62±3 | 61±4 | |

(Spalte 1)

Beispiel 7

Unter den Bedingungen des Beispiels 2 wurden stündlich 19 ml eines Gemisches, enthaltend 7% 1,2-Dichlorbenzol, 77% 1,2,4-Trichlorbenzol und 15% 1,2,3-Trichlorbenzol, umgesetzt. Nach einer Laufzeit von 111 Stunden wurde folgendes repräsentatives Produktgemisch analysiert:

| | |
|---|---|
| Benzol | 1,6 % |
| Chlorbenzol | 6,9 % |
| 1,2-Dichlorbenzol | 34,9 % |
| 1,3-Dichlorbenzol | 4,5 % |
| 1,4-Dichlorbenzol | 2,2 % |
| 1,2,4-Trichlorbenzol | 39,7 % |
| 1,2,3-Trichlorbenzol | 10,2 % |

Beispiel 8

Ein Gemisch aus 80% 1,2,4-Trichlorbenzol, 12% 1,2,4,5-Tetrachlorbenzol und 8% 1,2,3,4-Tetrachlorbenzol wurde bei 300°C an 1% Pt/1% MgO/1% KOH/LiAl-Spinell mit 4 l $H_2$/h umgesetzt. Nach 20 Stunden wurde folgendes Produkt gemisch erhalten.

| | |
|---|---|
| Benzol | 1,8 % |
| Chlorbenzol | 8,6 % |
| 1,2-Dichlorbenzol | 30,7 % |
| 1,3-Dichlorbenzol | 6,0 % |
| 1,4-Dichlorbenzol | 3,4 % |
| 1,2,4-Trichlorbenzol | 38,6 % |
| 1,2,3-Trichlorbenzol | 3,1 % |
| 1,2,4,5-Tetrachlorbenzol | 4,9 % |
| 1,2,3,4-Tetrachlorbenzol | 2,9 %. |

**Patentansprüche**

1. Verfahren zur Herstellung von 1,2-Dichlorbenzol, dadurch gekennzeichnet, daß man 1,2,4-Trichlorbenzol als Einsatzmaterial in der Gasphase bei einer Temperatur vom Siedepunkt des Einsatzmaterials bis 400°C mit Wasserstoff an einem Platin/Al-Spinell-Katalysator umsetzt.

2. Verfahren nach Anspruch 1, dadurch gekennzeichnet, daß 1,2,4-Trichlorbenzol im Gemisch mit anderen Polychlorbenzolen eingesetzt wird.

3. Verfahren nach Anspruch 2, dadurch gekennzeichnet, daß als Polychlorbenzole solche aus der Gruppe 1,2,3-Trichlorbenzol, 1,2,3,4-Tetrachlorbenzol und 1,2,4,5-Tetrachlorbenzol eingesetzt werden.

4. Verfahren nach Anspruch 1, dadurch gekennzeichnet, daß ein Gemisch mit einem Gehalt an 1,2,4-Trichlorbenzol eingesetzt wird, das aus der Chlorierung von 1,4-Dichlorbenzol entstanden ist.

5. Verfahren nach Anspruch 1, dadurch gekennzeichnet, daß bei 250 bis 350°C gearbeitet wird.

6. Verfahren nach Anspruch 1, dadurch gekennzeichnet, daß 0,5 bis 5 Mol Wasserstoff, bevorzugt 0,9 bis 2 Mol Wasserstoff, pro Äquivalent von zu entfernendem Chlor eingesetzt werden.

7. Verfahren nach Anspruch 1, dadurch gekennzeichnet, daß der Katalysatorträger LiAl-Spinell ist.

8. Verfahren nach Anspruch 1, dadurch gekennzeichnet, daß der Katalysator 0,1 bis 5 Gew.-% Platin, bezogen auf sein Gesamtgewicht, enthält.

9. Verfahren nach Anspruch 1, dadurch gekennzeichnet, daß der Katalysator mit Erdalkalimetall(hydr)oxid dotiert ist.

10. Verfahren nach Anspruch 9, dadurch gekennzeichnet, daß der Katalysator mit Magnesium(hydr)oxid dotiert ist.


**Claims**

1. Process for preparing 1,2-dichlorobenzene, characterised in that 1,2,4-trichlorobenzene is reacted as the starting material in the gas phase at a temperature from the boiling point of the starting material up to 400°C with hydrogen over a platinum/spinel catalyst.

2. Process according to Claim 1, characterised in that 1,2,4-trichlorobenzene is used as a mixture with other polychlorobenzenes.

3. Process according to Claim 2, characterised in that the polychlorobenzenes used are those from the group consisting of 1,2,3-trichlorobenzene, 1,2,3,4-tetrachlorobenzene and 1,2,4,5-tetrachlorobenzene.

4. Process according to Claim 1, characterised in that a mixture containing 1,2,4-trichlorobenzene is used which originated from the chlorination of 1,4-dichlorobenzene.

5. Process according to Claim 1, characterised in that it is carried out at 250 to 350°C.

6. Process according to Claim 1, characterised in that 0.5 to 5 mol of hydrogen, preferably 0.9 to 2 mol of hydrogen, are used per equivalent of chlorine to be removed.

7. Process according to Claim 1, characterised in that the catalyst carrier is LiAl spinel.

8. Process according to Claim 1, characterised in that the catalyst contains 0.1 to 5% by weight of platinum, based on its total weight.

9. Process according to Claim 1, characterised in that the catalyst is doped with an alkaline earth metal (hydr)oxide.

10. Process according to Claim 9, characterised in that the catalyst is doped with magnesium (hydr)oxide.


**Revendications**

1. Procédé de production de 1,2-dichlorobenzène, caractérisé en ce qu'on fait réagir le 1,2,4-trichlorobenzène comme charge en phase gazeuse à une température allant du point d'ébullition de la charge jusqu'à 400°C avec de l'hydrogène sur un catalyseur platine/spinelle d'aluminium.

2. Procédé suivant la revendication 1, caractérisé en ce qu'on utilise le 1,2,4-trichlorobenzène en mélange avec d'autres benzènes polychlorés.

3. Procédé suivant la revendication 2, caractérisé en ce qu'on utilise comme benzènes polychlorés des polychlorobenzènes du groupe du 1,2,3-trichlorobenzène, du 1,2,3,4-tétrachlorobenzène et du 1,2,4,5-tétrachlorobenzène.

4. Procédé suivant la revendication 1, caractérisé en ce qu'on utilise un mélange ayant une teneur en 1,2,4-trichlorobenzène obtenu par chloration du 1,4-dichlorobenzène.

5. Procédé suivant la revendication 1, caractérisé en ce qu'on opère à une température de 250 à 350°C.

6. Procédé suivant la revendication 1, caractérisé en ce qu'on utilise 0,5 à 5 moles d'hydrogène, de préférence 0,9 à 2 moles d'hydrogène, par équivalent de chlore à éliminer.

7. Procédé suivant la revendication 1, caractérisé en ce que le support du catalyseur est un spinelle de LiAl.

8. Procédé suivant la revendication 1, caractérisé en ce que le catalyseur contient 0,1 à 5% en poids de platine, par rapport à son poids total.

9. Procédé suivant la revendication 1, caractérisé en ce que le catalyseur est dopé avec un (hydr)oxyde de métal alcalino-terreux.

10. Procédé suivant la revendication 1, caractérisé en ce que le catalyseur est dopé à l'(hydr)oxyde de magnésium.